(19) Europäisches Patentamt
European Patent Office
Office européen des brevets

(11) EP 1 603 866 B1

(12) **EUROPEAN PATENT SPECIFICATION**

(45) Date of publication and mention of the grant of the patent:
**29.04.2009 Bulletin 2009/18**

(21) Application number: **02808315.2**

(22) Date of filing: **26.12.2002**

(51) Int Cl.:
***C07C 303/40*** (2006.01) ***C07C 303/42*** (2006.01)
***C07C 311/37*** (2006.01)

(86) International application number:
**PCT/IN2002/000244**

(87) International publication number:
**WO 2004/058694 (15.07.2004 Gazette 2004/29)**

(54) **A PROCESS FOR THE PREPARATION OF ENANTIOMERICALLY PURE (R) OR (S)-5-(2-AMINOPROPYL)-2-METHOXYBENZENESULFONAMIDE**

VERFAHREN ZUR ZUBEREITUNG VON ENANTIOMERISCH REINEM (R) ODER (S)-5-(2-AMINOPROPYL)-2-METHOXYBENZENSULFONAMID

PROCEDE DE PREPARATION DE (R) OU (S)-5-(2-AMINOPROPYL)-2-METHOXYBENZENESULFONAMIDE ENANTIOMERIQUEMENT PUR

(84) Designated Contracting States:
**AT BE BG CH CY CZ DE DK EE ES FI FR GB GR IE IT LI LU MC NL PT SE SI SK TR**

(43) Date of publication of application:
**14.12.2005 Bulletin 2005/50**

(73) Proprietor: **CADILA HEALTHCARE LIMITED**
**Amedadabad 380 015**
**Gujarat (IN)**

(72) Inventors:
- **DUBEY, Sushil K., c/o Cadila Healthcare Limited Ahmedabad 380 015, Gujarat (IN)**
- **PATEL, Dharmeshkumar A. Cadila Healthcare Limited Ahmedabad 380 015, Gujarat (IN)**
- **PATEL, Dhimant J., c/o Cadila Healthcare Limited Ahmedabad 380 015, Gujarat (IN)**
- **RUPAPARA, Mahesh L., c/o Cadila Healthcare Limited Ahmedabad 380 015, Gujarat (IN)**
- **PANDITA, Kanwal, c/o Cadila Healthcare Limited Ahmedabad 380 015, Gujarat (IN)**
- **PATEL, Ramanbhai c/o Cadila Healthcare Limited Ahmedabad 380 015, Gujarat (IN)**

(74) Representative: **Spencer, Michael David et al**
**Bromhead Johnson**
**19 Buckingham Street**
**London**
**WC2 6EF (GB)**

(56) References cited:
**EP-A- 0 257 787** **US-A- 2 233 823**

- **R.A. SMITH ET AL: J. MED. CHEM., vol. 31, no. 8, 1988, pages 1558-1566, XP002239168**
- **DATABASE CAPLUS [Online] CHEMICAL ABSTRACTS SERVICE, COLUMBUS, OHIO, US; retrieved from STN Database accession no. 1996: 339542 XP002239170 & J. ZHAO ET AL: ZHEJIANG GONGYE DAXUE XUEBAO BIANJIBU, vol. 24, no. 1, pages 33-37,**
- **E.R. SHEPARD ET AL: J. AM. CHEM. SOC., vol. 74, no. 18, 1952, pages 4611-4615, XP002239169**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 603 866 B1

## Description

The present invention relates to a process for the preparation of enantiomerically pure (R) or (S)-5-(2- aminopropyl)-2-methoxybenzenesulfonamide by resolution of (R, S)-5-(2-aminopropyl)-2-methoxybenzenesulfonamide with D-i.e. (2S, 3S) or L-i.e. (2R, 3R)-tartaric acid respectively. The optically pure (R)-(-)-5-(2-aminopropyl) -2-methoxybenzenesulfonaminde of formula I is a key intermediate for the preparation of Tamsulosin of formula II (EP-34432, US-4703063) useful in the treatment of patients with symptomatic benign prostatic hyperplasia (BPH).

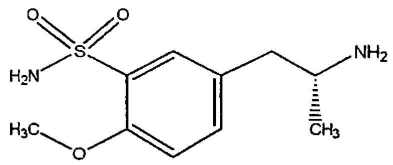

I

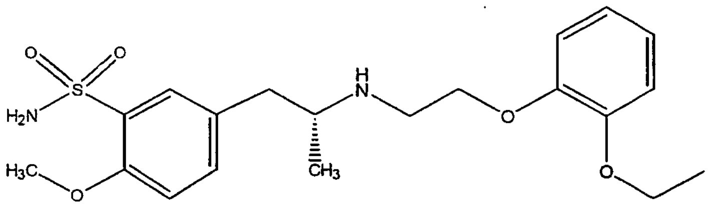

II

## BACKGROUND OF THE INVENTION

European Patent 257787 describes a method for the preparation of optically pure (R)-(-)-5-(2-aminopropyl)-2-methoxybenzenesulfonamide based on the principle of diastereomeric separation. Thus 5-acetonyl-2-methoxybenzenesulfonamide (III) as in scheme-1 is reacted with an optically active benzylic amine (IV) under reducing conditions to obtain a diastereomeric mixture (V) (RR: SR) in varying ratio depending upon the reducing agent employed. The material thus obtained is subjected to the following operations:

(i) suspension in a solvent, (ii) heating for a specified time, (iii) cooling, (iv) filtration. This cycle of operations is repeated (at least four times) till an optical purity of 98.0 % of VI is achieved.

Compound VI of 98% optical purity is again mixed with water & another solvent such as acetone or acetonitrile. The mixture is heated to reflux, cooled & filtered. The crystals obtained are subjected to repeated cycles of mixing with the solvent, refluxing, cooling & filtration (at least three times) until desired optical purity of >99.5% is obtained for Compound VII (Scheme - 1).

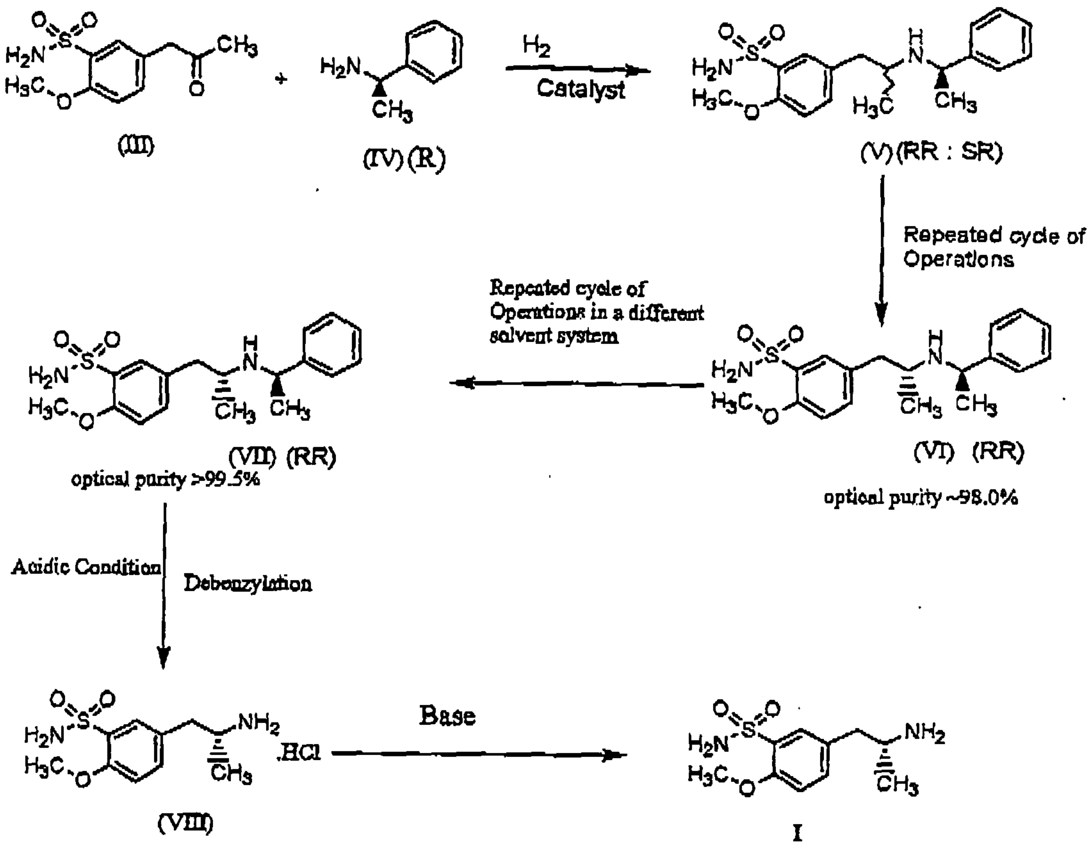


**Scheme :1**

Optically pure diastereomer VII is debenzylated under acidic conditions to obtain R-(-)-5-(2-aminopropyl)-2-methoxybenzenesulphonamide as hydrochloride salt (VIII), which is isolated and treated with a base to obtain desired compound I.

US -A- 4703063 relates to novel sulphamoyl-substituted phenethylamine derivatives and the acid addition salts thereof, and especially, to novel sulphamoyl-substituted phenethylamine derivatives and the acid addition salts thereof which exhibit a strong alpha-adrenergic blocking action and are useful as an antihypertensive agent and a treating agent for congestive heart failure.

A process for the resolution of allenic amines is disclosed in J. Med. Chem. 1988, 31(8), 1558-1566 using different resolution agents such as α-(+)-methylbenzyl amine and tartaric acid. Zhejiang Gongye Daxue Xuebao (1996), 24(1), 33-37 discloses a process for the resolution of DL-1-(aminophenyl)-2-methylaminopropane by using D-tartaric acid in an ethanol-water mixture.

## OBJECTS OF THE INVENTION

It is evident from above that although the prior art looks conceptually very good, practically it is very cumbersome and time consuming. One will have to carry out a number of repeated operations as well as analysis to obtain optically pure I. Therefore, the above mentioned process is not a suitable process for large-scale production.

Accordingly, it is an aim of the present invention to provide a plant friendly yet inexpensive process for the manufacture of R-(-)-5-(2-aminopropyl)-2-methoxybenzenesulphonamide (I). This aim of the present invention can only be achieved, if one can devise a suitable diastereomeric salt of I whose differential solubility properties could be exploited in a suitable solvent system at an appropriate temperature range to obtain desired optically pure (R)-(-)-5-(2-aminopropyl)-2-methoxybenzenesulphonamide (I) in the minimum possible operations.

## DETAILED DESCRIPTION

Accordingly, the present invention provides a process for the manufacture of optically pure (R) or (S)-5-(2-aminopropyl)-2-methoxybenzenesulphonamide (I), as described in claims 1 to 9, which comprises resolving (R,S)-5-(2-aminopropyl)-2-methoxybenzene-sulphonamide with D- or L-tartaric acid to form a mixture of diastereomeric salts, separating the diastereomeric salts in two stage process in a mixture of solvent systems of the kind such as described at a specified temperature range and contacting the individual salts so separated with base of the kind such as herein described to certain pH range such as herein described to provide said (R)-(-)-5-(2-aminopropyl)-2-methoxybenzenesulphonamide (I) in optically pure form (Scheme-2).

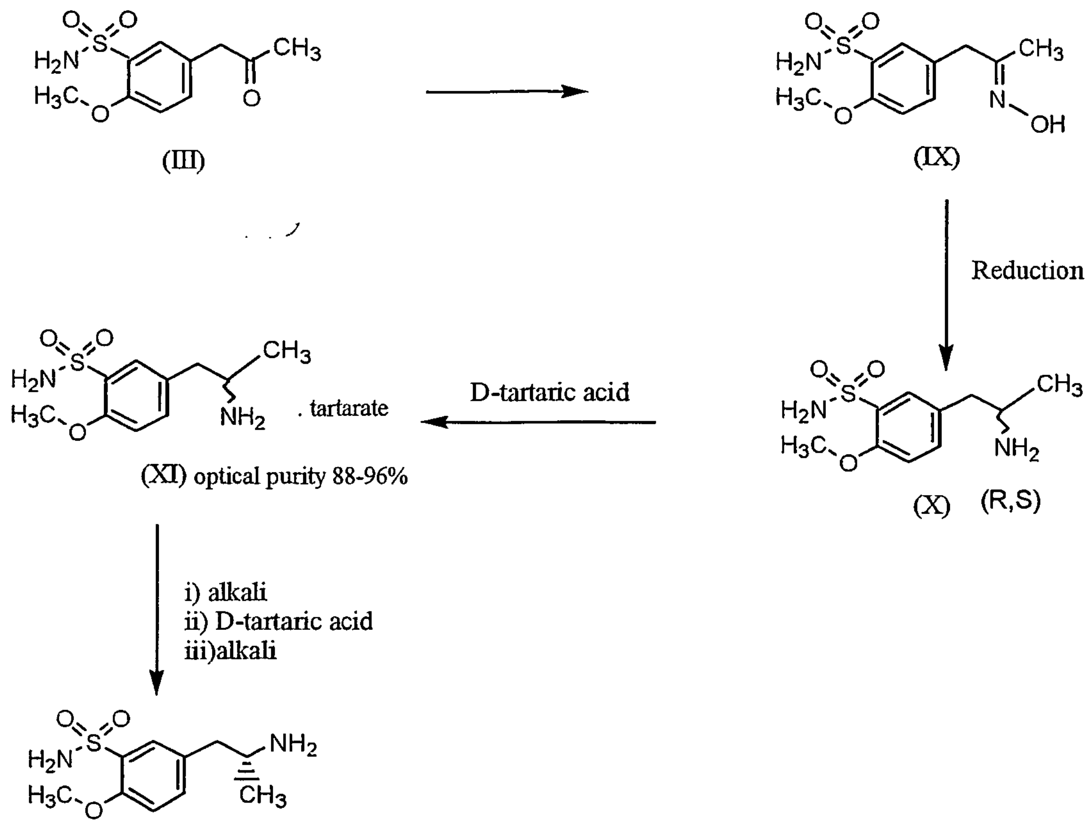


(III) (IX) (X) (R,S) (XI) optical purity 88-96%

I

optical purity >99.5%

**Scheme :2**

(R, S)-5-(2- aminopropyl)-2-methoxybenzenesulfonamide can be prepared by any known procedure but is preferably prepared by a two step procedure (Scheme-2) starting from 5-acetonyl-2-methoxybenzenesulfonamide (III) converting its keto functional group into oxime (IX) & reducing it with hydrogen under catalytic conditions to obtain the racemic amino compound (X).

Optical purity of more than 99.5% is achieved through two stage procedure. In the first stage racemic 5-(2-aminopropyl)-2-methoxybenzenesulfonamide (X) is reacted with D-tartaric acid to form the salt (XI) having a melting point between 189-195°C Salt obtained is cleaved with alkali and pH is adjusted between 9.5-10. Substantially resolved amine is obtained as a crystalline solid. This is again treated with D-tartaric acid followed by desalting to obtain optically pure R (-)-5-(2-aminopropyl)-2-methoxybenzenesulfonamide (I).

Preferably, the ratio of (R, S)-5-(2- aminopropyl)-2-methoxybenzenesulfonamide to the D (-) tartaric acid is 1: 1 1 to 1:1.1 mole. The diastereomeric salt formation as well as resolution is preferably carried out in the same solvent system, but these operations can also be performed in two different solvents.

It has been observed that the resolution of 5-(2-aminopropyl)-2-methoxybenzenesulfonamide tartarate is largely governed by the polarity of the solvent system used. The solvent system preferred is a combination of alcoholic solvents such as methanol, ethanol, propanol having 5-20% (v/v) of polar solvents such as amidic solvents e.g. dimethylformamide, N-methyl-2-pyrrolidone; dimethylsulfoxide or water. Though water alone can also be used for salt formation and resolution

at room temperature, however in order to obtain optimum yield and optical purity, it's used in combination with alcoholic solvents.

Temperature plays a major role in obtaining the optically pure R- (-)-5-(2-aminopropyl)-2-methoxybenzenesulfonamide. It has been observed that if the resolution is carried out at 50-70°C, the desired product is obtained in a higher optical purity. Temperature between 60- 65°C provides the best results and is therefore preferable.

The reaction time may vary between 4 to 26 hrs after the addition of the amine to the tartaric acid; however under optimal reaction conditions, reaction time of 6 to 8 hrs is preferred to obtain the optimum optical purity as well as yield.

The separated solid salt (R amine: D acid) from the reaction mass is isolated by filtration. Optically purified diastereomeric salt is treated with a base preferably sodium hydroxide to bring pH 9.5 - 10 to obtain free R- (-)-5-(2-aminopropyl)-2-methoxybenzenesulfonamide.

From the mother liquor obtained from second stage filtration, 5-(2-aminopropyl)-2-methoxybenzenesulfonamide is recovered in ~ 90% optical purity and is used in next cycle of resolution to enhance the productivity. It can also be resolved separately if so desired.

The resolving agent D-tartaric acid can be recovered from the aqueous part by usual methods known in the literature and can be recycled for resolution.

Besides D-tartaric acid, L-tartaric acid can also be used as a resolving agent. The resolution of (R, S)-5-(2-aminopropyl)-2-methoxybenzenesulfonamide using L-tartaric acid is carried out in the same fashion. However in this case, (2S, 3S,S)- diastereomeric salt separates out from the reaction mixture which after desalting gives the S- (+)-5-(2-aminopropyl)-2-methoxybenzenesulfonamide.

The present invention is further described in greater detail as illustrated in nonlimiting examples.

Example : 1.

**5-(2-Hydroxyiminopropyl)-2-methoxybenzenesulfonamide**

A mixture of 55 gm 5-acetonyl-2-methoxybenzenesulfonamide, 23.6 gm hydroxylamine hydrochloride, 35.4 gm triethylamine and 275 ml of methanol was stirred at room temperature for 5 hours. Then methanol was distilled out and after distillation 275 ml water was added to the reaction mass. The reaction mixture was stirred for 1 hour at room temperature and separated solid was filtered. 49.5 gm 5-(2-hydroxyiminopropyl)-2-methoxy benzenesulfonamide was obtained.

Melting point: 193-4°C ; $^1$H NMR: 1.7 δ ($-CH_3$), 3.5 δ ($-CH_2-$), 3.95 δ ($-O-CH_3$), 7.1, 7.4, 7.7 δ (aromatic-H) ; m/e : 259.1 $(M+H)^+$.

Example : 2.

**5-(2-aminopropyl)-2-methoxybenzenesulfonamide**

A mixture of 30 gm 5-(2-hydroxyiminopropyl)-2-methoxybenzene sulfonamide, 10.5 gm Raney nickel (wet) and 450 ml methanol was shaken in a Parr apparatus at room temperature under 4 kg hydrogen pressure for 8 hours. At the end of reaction, Raney nickel was filtered off. Filtrate was distilled out to obtain 27.6 gm 5-(2-aminopropyl)-2-methoxybenzenesulfonamide.

Melting point: 180°C ; $^1$H NMR: 0.9 δ ($-CH_3$), 2.9 δ (-CH-), 2.5 δ ($-CH_2-$), 3.8 δ ($-O-CH_3$), 7.1, 7.3, 7.5 δ (aromatic-H) ; m/e : 245.4 $(M+H)^+$.

Example : 3.

**R- (-)-5-(2-aminopropyl)-2-methoxybenzenesulfonamide tartarate**

50 gm 5-(2-aminopropyl)-2-methoxybenzenesulfonamide was dissolved in a solvent mixture of 500 ml of methanol and water (9.5: 0.5 v/v) on heating. 33.8 gm D (-) tartaric acid was added slowly at 65°C in the reaction mixture, maintained the temperature for 6 hours. The crystals were collected by filtration, washed with methanol and dried to provide 30.40 gm tartarate salt of R-(-)-5-(2-aminopropyl)-2-methoxybenzenesulfonamide tartarate.

Meltin, $[\alpha]^{25}_{D}$ (c = 1.0 , $H_2O$) : -19.4°

Example : 4.

**R- (-)-5-(2-aminopropyl)-2-methoxybenzenesulfonamide tartarate**

25 gm of 5-(2-aminopropyl)-2-methoxybenzenesulfonamide was dissolved in 250 ml of a solvent mixture of methanol and dimethyl sulfoxide (8.0: 2.0 v/v) on heating. 16.9 gm D (-) tartaric acid was added 60 °C, cooled it to 50°C and maintained it for 26 hours. The crystals were collected by filtration, washed with methanol and dried to provide 7.6 gm tartarate salt of R- (-)-5-(2-aminopropyl)-2-methoxy benzene sulfonamide.

Melting point: 196-7 °C (d). $[\alpha]_D^{25}$ (c = 1.0, $H_2O$) : -20.6°

Example : 5.

**R- (-)-5-(2-aminopropyl)-2-methoxybenzenesulfonamide tartarate**

250 gm of R- (-)-5-(2-aminopropyl)-2-methoxybenzenesulfonamide was dissolved in 2125 ml of methanol and 425 ml dimethyl formamide on heating. 169 gm D (-) tartaric acid was added slowly at 60 °C, maintained temperature for 6 hours. The crystals were filtered off. The crystals, which were separated out (wet), were taken in 750 ml of methanol and stirred for half an hour. The solid was filtered off and washed with methanol, thereby affording 193.3 gm. tartarate salt of R- (-)-5-(2-aminopropyl)-2-methoxybenzenesulfonamide.

Melting point: 193-4 °C(d); $[\alpha]_D^{25}$ (c = 1.0 , $H_2O$) : -19.16°

Example: 6

**R- (-)-5-(2-aminopropyl)-2-methoxybenzenesulfonamide tartarate**

25 gm of 5-(2-aminopropyl)-2-methoxybenzenesulfonamide was dissolved in 250 ml of solvent mixture of isopropyl alcohol and water (8 : 2 v/v) on heating 16.9 gm D (-) tartaric acid was added at 70 °C, maintained it for 6 hours. The crystals were collected by filtration, washed with isopropyl alcohol and dried to provide 17.9gm tartarate salt of R-(-)-5-(2-aminopropyl)-2-methoxy- benzenesulfonamide.

Melting point: 189-90 °C(d); $[\alpha]_D^{25}$ (c = 1.0 , $H_2O$) : -17.4°

Example: 7

**R- (-)-5-(2-aminopropyl)-2-methoxybenzenesulfonamide tartarate**

25 gm of 5-(2-aminopropyl)-2-methoxybenzenesulfonamide was dissolved in 250 ml solvent mixture of methanol and dimethyl sulfoxide (9.5 : 0.5 v/v) on heating. 16.9 gm D (-) tartaric acid was added at 60 °C, maintained it for 6 hours. The crystals were collected by filtration, washed with methanol and dried to provide 18.9 gm tartarate salt of R-(-)-5-(2-aminopropyl)-2-methoxy-benzenesulfonamide.
Melting point: 188-9 °C(d);

Example: 8

**R-(-)-5-(2-aminopropyl)-2-methoxybenzenesulfonamide tartarate**

25 gm 5-(2-aminopropyl)-2-methoxybenzenesulfonamide was dissolved in 250 ml solvent mixture of methanol and dimethyl sulfoxide (9 : 1 v/v) on heating. 16.9 gm D (-) tartaric acid was added at 60 °C, maintained it for 6 hours. The crystals were collected by filtration, washed with methanol and dried to provide 15.1 gm tartarate salt of R-(-)-5-(2-aminopropyl)-2-methoxybenzenesulfonamide.

Melting point: 193-4°C(d); $[\alpha]_D^{25}$ (c = 1.0, $H_2O$) : -18.70°

Example: 9

**R-(-)-5-(2-aminopropyl)-2-methoxybenzenesulfonamide tartarate**

25 gm 5-(2-aminopropyl)-2-methoxybenzenesulfonamide was dissolved in 250 ml solvent mixture of methanol and N-methyl 2-pyrrolidone (9.0 : 1.0 v/v) on heating. 16.9 gm D (-) tartaric acid was added at 60 °C, maintained it for 6 hours. The crystals were collected by filtration, washed with methanol and dried to provide 15.0 gm tartarate salt of R-(-)-5-(2-aminopropyl)-2-methoxy-benzenesulfonamide.

Melting point: 192-3 °C(d); $[\alpha]^{25}_{D}$ (c = 1.0, $H_2O$) : -18.49°

Example: 10

**R- (-)-5-(2-aminopropyl)-2-methoxybenzenesulfonamide tartarate**

25 gm 5-(2-aminopropyl)-2-methoxybenzenesulfonamide was dissolved in 250 ml solvent mixture of methanol and N-methyl 2-pyrrolidone (8.0 : 2.0 v/v) on heating. 16.9 gm D (-) tartaric acid was added at 60 °C, maintained it for 6 hours. The crystals were collected by filtration, washed with methanol and dried to provide 13.3 gm tartarate salt of R-(-)-5-(2-aminopropyl)-2-methoxybenzenesulfonamide.

Melting point: 193-4°C(d); $[\alpha]^{25}_{D}$ (c = 1.0, $H_2O$): -18.57°

Example: 11

**R-(-)-5-(2-aminopropyl)-2-methoxybenzenesulfonamide tartarate**

25 gm 5-(2-aminopropyl)-2-methoxybenzenesulfonamide was dissolved in 375 ml solvent mixture of methanol and water (9.5 : 0.5 v/v) on heating. 16.9 gm D (-) tartaric acid was added at 60 °C, maintained it for 14 hours. The crystals were collected by filtration, washed with methanol and dried to provide 10.4 gm tartarate salt of R-(-)-5-(2-aminopropyl)-2-methoxybenzenesulfonamide.

Melting point: 196-7°C (d); $[\alpha]^{25}_{D}$ (c = 1.0, $H_2O$) : -20.29°

Example: 12

**R- (-)-5-(2-aminopropyl)-2-methoxybenzenesulfonamide tartarate**

25 gm 5-(2-aminopropyl)-2-methoxybenzenesulfonamide was dissolved in 375 ml methanol and 37.5 ml N, N-dimethylformamide on heating. 16.9 gm D (-) tartaric acid was added at 60 °C, maintained it for 14.5 hours. The crystals were collected by filtration, washed with methanol and dried to provide 11.7 gm tartarate salt of R- (-)-5-(2-aminopropyl)-2-methoxy benzenesulfonamide.

Melting point: 197-8°C(d); $[\alpha]^{25}_{D}$ (c = 1.0 , $H_2O$) : -20.7 °

Example: 13

**R- (-)-5-(2-aminopropyl)-2-methoxybenzenesulfonamide tartarate**

25 gm 5-(2-aminopropyl)-2-methoxy benzenesulfonamide was dissolved in 250 ml methanol and 12.5 ml N, N-dimethylformamide on heating. 16.9 gm D(-) tartaric acid was added at 60 °C, maintained the temperature for 6 hours. The crystals were collected by filtration, washed with methanol and dried to provide 16.1 gm tartarate salt of R-(-)-5-(2-aminopropyl)-2-methoxybenzenesulfonamide.

Melting point: 191-2°C (d); $[\alpha]^{25}_{D}$ (c = 1.0, $H_2O$) : -19.11°

Example: 14

**R-(-)-5-(2-aminopropyl)-2-methoxybenzenesulfonamide tartarate**

25 gm 5-(2-aminopropyl)-2-methoxybenzenesulfonamide was dissolved in 175 ml solvent mixture of methanol and water (9 : 1 v/v) on heating. 16.9 gm D(-) tartaric acid was added at 60°C, maintained the temperature at 65°C for 14 hours. The crystals were collected by filtration, washed with methanol and dried to provide 15.4 gm tartarate salt of R-(-)-5-(2-aminopropyl)-2-methoxy- benzenesulfonamide.

Melting point: 194-5 °C(d); $[\alpha]^{25}_{D}$ (c = 1.0 , $H_2O$): -19.12 °

Example: 15

**R- (-)-5-(2-aminopropyl)-2-methoxybenzenesulfonamide**

To 75gm tartarate salt (as obtained in example: 5: m.p.:193-4°C) of R-(-)-5-(2-aminopropyl)-2-methoxybenzene sulfonamide in 75 ml water was added 40% sodium hydroxide solution, so as to adjust pH between 9.5 - 10. Reaction mixture was stirred for 1 hour at room temperature. 38.7 gm of R-(-)-5-(2-aminopropyl)-2-methoxybenzenesulfonamide was collected after filtration.

Melting point: 170-1°C; $[\alpha]^{25}_{D}$ (c = 1.0, $H_2O$): -14.49°

Example: 16

**R-(-)-5-(2-aminopropyl)-2-methoxybenzenesulfonamide tartarate**

25 gm of R-(-)-5-(2-aminopropyl)-2-methoxybenzenesulfonamide (m.p.:170°C) was dissolved in solvent mixture of 250 ml methanol and 50 ml N,N-dimethylformamide on heating. 16.9 gm D (-) tartaric acid was added at 60 °C, maintained the temperature for 6 hours. The crystals formed were collected by filtration, washed with methanol. The crystals, which were collected out (wet), were stirred for ½ an hour with 75 ml methanol. The solid was filtered off and washed with methanol, affording 32.7 gm tartarate salt of R-(-)-5-(2-aminopropyl)-2-methoxybenzenesulfonamide.

Melting point: 198 °C(d); $[\alpha]^{25}_{D}$ (c = 1.0, $H_2O$) : -20.5 °

Example: 17

**R-(-)-5-(2-aminopropyl)-2-methoxybenzenesulfonamide tartarate**

10 gm of R-(-)-5-(2-aminopropyl)-2-methoxybenzenesulfonamide (m.p.=170°C ) was dissolved in 100 ml solvent mixture of methanol and water (9.3 : 0.7 v/v) on heating, 6.8 gm D(-) tartaric acid was added at 65 °C, maintained the temperature for 6 hours. The crystals formed were collected by filtration, washed with methanol and dried to provide 8.9 gm tartarate salt of R-(-)-5-(2-aminopropyl)-2-methoxybenzenesulfonamide.

Melting point: 198 °C(d); $[\alpha]^{25}_{D}$ (c = 1.0, $H_2O$) : -20.6 °

Example: 18

**R-(-)-5-(2-aminopropyl)-2-methoxybenzenesulfonamide tartarate**

10 gm of R-(-)-5-(2-aminopropyl)-2-methoxybenzenesulfonamide (m.p.: 172°C)was dissolved in 100 ml a solvent mixture of methanol and water (9 : 1 v/v) on heating, 6.8 gm D(-) tartaric acid was added at 65°C, maintain it for 8 hours. Crystals formed were collected by filtration washed with methanol and dried to provide 10.9 gm tartarate salt of R-(-)-5-(2-aminopropyl)-2-methoxy benzenesulfonamide.

Melting point: 198°C(d); $[\alpha]^{25}_{D}$ (c = 1.0 , $H_2O$) : -20.6 °

Example: 19

**R- (-)-5-(2-aminopropyl)-2-methoxybenzenesulfonamide**

To 10 gm tartarate salt (as obtained in example 16 ) of R-(-)-5-(2-aminopropyl)-2-methoxybenzene sulfonamide in 10 ml water was added 40% sodium hydroxide solution, so as to adjust pH between 9.5 - 10. Reaction mixture was stirred for 1 hour at room temperature. 6.0 gm of R- (-)-5-(2-aminopropyl)-2-methoxybenzenesulfonamide was collected after filtration. Melting point: 16: $[\alpha]_{D}^{23}$ c = 1.07, Methanol) : -17.11° (lit: -17.3°)

**Claims**

1. A process for the manufacture of optically pure (R) or (S) -5-(2-aminopropyl)-2-methoxybenzenesulphonamide comprising, resolving (R, S) -5-(2-aminopropyl)-2-methoxybenzenesulphonamide with (D)- or (L)-tartaric acid in a suitable solvent system to form a mixture of diastereomeric salts, separating the diastereomeric salts in any known manner and contacting any of the individual salts so separated with base to provide R-(-)-5-(2-aminopropyl)-2-methoxybenzenesulphonamide or S-(+)-5-(2-aminopropyl)-2-methoxybenzenesulphonamide, wherein the suitable solvent system comprises a polar solvent and an alcoholic solvent in a ratio varying from 5 to 20% (v/v), and the resolution is carried out in a temperature range of 50-70°C.

2. A process according to claim 1, **characterised in that** the ratio of (R, S) -5-(2-aminopropyl)-2-methoxybenzenesulphonamide to tartaric acid is in the range of 1:1 to 1:1.1.

3. A process according to claim 1 or claim 2, **characterised in that** the resolution is carried out in two stages in the presence of a solvent system consisting of alcoholic solvents coupled with varying ratios of polar solvents.

4. A process according to any preceding claim, **characterised in that** the polar solvent is an amidic solvent, dimethylsulfoxide or water.

5. A process according to any preceding claim, **characterised in that** the resolution is carried out at a temperature in the range of 60-65°C.

6. A process according to any preceding claim, **characterised in that** the reaction time is between 4 to 26 hours.

7. A process according to any preceding claim, **characterised in that** the solvent system used for separating the diastereomeric salts to individual salts is selected from the group consisting of one or more of methanol, ethyl alcohol, propyl alcohol and one or more of water, dimethylformamide, N-methyl-2-pyrrolidonc, dimethylsulphoxide.

8. A process according to any preceding claim, **characterised in that** the base is sodium hydroxide and the pH for isolation of free base is 9.5-10.

9. A process for the manufacture of optically pure (R) -5-(2-aminopropyl)-2-methoxybenzenesulphonamide comprising the steps of:

   i) converting the keto functional group of 5-acetonyl-2-methoxybenzenesulphonamide into an oxime group;
   ii) reducing the oxime group with hydrogen under catalytic conditions to obtain racemic (R,S) -5-(2-aminopropyl)-2-methoxybenzenesulphonamide;
   iii) reacting racemic (R,S) -5-(2-aminopropyl)-2-methoxybenzenesulphonamide with D-tartaric acid to form a salt;
   iv) cleaving the salt with alkali metal hydroxide and adjusting the pH to between 9.5 and 10 to form a resolved amine as a crystalline solid;
   v) treating the amine again with (D)-tartaric acid in a suitable solvent system comprised of a polar solvent and an alcoholic solvent in a ratio varying from 5 to 20% (v/v) in a temperature range of 50-70°C, followed by desalting to obtain optically pure (R)-(-)-5-(2-aminopropyl)-2-methoxybenzenesulphonamide.

## Patentansprüche

1. Verfahren zur Herstellung von optisch reinem (R)- oder (S)-5-(2-Amino propyl)-2-methoxybenzolsulfonamid, umfassend das Trennen von (R, S)-5-(2-Amino propyl)-2-methoxybenzolsulfonamid mit (D)- oder (L)-Weinsäure in einem geeigneten Lösungsmittelsystem, um eine Mischung von diastereomeren Salzen zu bilden, Abtrennen der diastereomeren Salze in irgendeiner bekannten Weise und in Kontakt bringen jedes beliebigen der derartig abgetrennten individuellen Salze , mit einer Base, um R-(-)-5-(2-Amino propyl)-2-methoxybenzolsulfonamid oder S-(+)-5-(2-Amino propyl)-2-methoxybenzolsulfonamid zu liefern, wobei das geeignete Lösungsmittelsystem ein polares Lösungsmittel und ein alkoholisches Lösungsmittel in einem Verhältnis enthält, das von 5 bis 20 % (Volumen/ Volumen) variiert, und das Trennen in einem Temperaturbereich von 50 bis 70°C durchgeführt wird.

2. Verfahren nach Anspruch 1, **dadurch gekennzeichnet, dass** das Verhältnis von (R,S)-5-(2-Aminopropyl)-2-methoxybenzolsulfonamid zu Weinsäure in dem Bereich von 1:1 bis 1:1,1 liegt.

3. Verfahren nach Anspruch 1 oder 2, **dadurch gekennzeichnet, dass** das Trennen in zwei Stufen in Gegenwart eines Lösungsmittelsystems durchgeführt wird, das aus alkoholischen Lösungsmitteln kombiniert mit variierenden Verhältnissen von polaren Lösungsmittel besteht.

4. Verfahren nach irgendeinem vorhergehenden Anspruch, **dadurch gekennzeichnet, dass** das polare Lösungsmittel ein amidisches Lösungsmittel, Dimethylsulfoxid oder Wasser ist.

5. Verfahren nach irgendeinem vorhergehenden Anspruch, **dadurch gekennzeichnet, dass** das Trennen bei einer Temperatur in dem Bereich von 60 bis 65°C durchgeführt wird.

6. Verfahren nach irgendeinem vorhergehenden Anspruch, **dadurch gekennzeichnet, dass** die Reaktionszeit zwischen 4 und 26 Stunden liegt.

7. Verfahren nach irgendeinem vorhergehenden Anspruch, **dadurch gekennzeichnet, dass** das Lösungsmittelsystem, das zum Auftrennen der diastereomeren Salze in individuelle Salze verwendet wird, aus der Gruppe ausgewählt wird, die aus einem oder mehreren von Methanol, Ethylalkohol und Propanol sowie ein oder mehreren von Wasser, Dimethylformamid, N-Methyl-2-pyrrolidon, Dimethylsulfoxid besteht.

8. Verfahren nach irgendeinem vorhergehenden Anspruch, **dadurch gekennzeichnet, dass** die Base Natriumhydroxid darstellt und der pH zur Isolierung der freien Basen 9,5 bis 10 beträgt.

9. Verfahren zur Herstellung von optisch reinem (R)-5-(2-Aminopropyl)-2-methoxybenzolsulfonamid, umfassend die Schritte:

   **i)** Umwandeln der keto-funktionellen Gruppe vom 5-Acetonyl-2-methoxybenzolsulfonamid in eine Oxim-Gruppe;
   **ii)** Reduzieren der Oxim-Gruppe mit Wasserstoff unter katalytischen Bedingungen, um racemisches (R,S)-5-(2-Aminopropyl)-2-methoxybenzolsulfonamid zu erhalten;
   **iii)** Umsetzen des racemischen (R,S)-5-(2-Aminopropyl)-2-methoxybenzolsulfonamids mit D-Weinsäure, um ein Salz zu bilden;
   **iv)** Spalten des Salzes mit Alkalimetalhydroxid und Einstellung des pH zwischen 9,5 und 10, um ein abgetrenntes Amin als kristallinen Feststoff zu erhalten;
   **v)** erneutes Behandeln des Amins mit (D)-Weinsäure in einem geeigneten Lösungsmittelsystem, das ein polares Lösungsmittel und ein alkoholisches Lösungsmittel in einem Verhältnis variierend von 5 bis 20 % (Volumen/ Volumen) enthält, in einem Temperaturbereich von 50 bis 60°C, gefolgt von einem Entsalzen, um optisch reines (R)-(-)-5-(2-Aminopropyl)-2-methoxybenzolsulfonamid zu erhalten.

## Revendications

1. Procédé de fabrication du (R)- ou (S)-5-(2-aminopropyl)-2-méthoxybenzènesulfonamide optiquement pur, comprenant la résolution du (R, S) -5-(2-aminopropyl)-2-méthoxybenzènesulfonamide par l'acide D- ou L-tartrique dans un système solvant approprié pour former un mélange de sels diastéréomères, la séparation des sels diastéréomères de n'importe quelle manière connue, et la mise en contact des sels individuels ainsi séparés avec la base pour fournir le R-(-)-5-(2-aminopropyl)-2-méthoxybenzènesulfonamide ou le S-(+)-5-(2-aminopropyl)-2-méthoxybenzènesulfonamide, le système solvant approprié comprenant un solvant polaire et un solvant alcoolique dans un rapport variant de 5 à 20% (v/v), et la résolution étant effectuée dans une plage de température de 50-70°C.

**2.** Procédé selon la revendication 1, **caractérisé par le fait que** le rapport du (R, S) -5-(2-aminopropyl)-2-méthoxybenzènesulfonamide à l'acide tartrique se situe dans la plage de 1 : 1 à 1 : 1,1.

**3.** Procédé selon la revendication 1 ou la revendication 2, **caractérisé par le fait que** la résolution est effectuée en deux stades en présence d'un système solvant consistant en solvants alcooliques couplés avec des rapports variables de solvants polaires.

**4.** Procédé selon l'une quelconque des revendications précédentes, **caractérisé par le fait que** le solvant polaire est un solvant amidique, le diméthylsulfoxyde ou l'eau.

**5.** Procédé selon l'une quelconque des revendications précédentes, **caractérisé par le fait que** la résolution est effectuée à une température se situant dans la plage 60-65°C.

**6.** Procédé selon l'une quelconque des revendications précédentes, **caractérisé par le fait que** le temps de réaction est entre 4 et 26 heures.

**7.** Procédé selon l'une quelconque des revendications précédentes, **caractérisé par le fait que** le système solvant utilisé pour séparer les sels diastéréomères des sels individuels est choisi dans le groupe constitué par un ou plusieurs parmi le méthanol, l'alcool éthylique, l'alcool propylique et un ou plusieurs parmi l'eau, le diméthylformamide, la N-méthyl-2-pyrrolidone, le diméthylsulfoxyde.

**8.** Procédé selon l'une quelconque des revendications précédentes, **caractérisé par le fait que** la base est l'hydroxyde de sodium et le pH pour l'isolement de la base libre est de 9,5-10.

**9.** Procédé de fabrication de (R)-5-(2-aminopropyl)-2-méthoxybenzènesulfonamide optiquement pur, comprenant les étapes consistant à :

i) convertir le groupe fonctionnel céto du 5-acétonyl-2-méthoxybenzènesulfonamide en un groupe oxime ;
ii) réduire le groupe oxime par l'hydrogène dans des conditions catalytiques pour obtenir le (R, S) -5-(2-aminopropyl)-2-méthoxybenzènesulfonamide racémique ;
iii) faire réagir le (R,S)-5-(2-aminopropyl)-2-méthoxybenzènesulfonamide racémique avec l'acide D-tartrique pour former un sel ;
iv) cliver le sel par un hydroxyde de métal alcalin et ajuster le pH entre 9,5 et 10 pour former une amine résolue en tant que solide cristallin ;
v) traiter l'amine à nouveau par l'acide D-tartrique dans un système solvant approprié composé d'un solvant polaire et d'un solvant alcoolique dans un rapport variant de 5 à 20% (v/v) dans une plage de température de 50-70°C, en faisant suivre par un dessalement pour obtenir le (R)-(-)-5-(2-aminopropyl)-2-méthoxybenzènesulfonamide optiquement pur.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 34432 A
- US 4703063 A
- EP 257787 A

### Non-patent literature cited in the description

- *J. Med. Chem.,* 1988, vol. 31 (8), 1558-1566
- *Zhejiang Gongye Daxue Xuebao,* 1996, vol. 24 (1), 33-37